# EUROPEAN PATENT APPLICATION

(11) **EP 4 667 482 A1**
(43) Date of publication of application: **24.12.2025**
(21) Application number: 24756139.2
(22) Date of filing: 06.02.2024
(51) Int. Cl.: C07K 5/062, C07K 5/06, A61K 38/00, A61K 38/05, A61P 7/02, A61P 9/10

(54) **SALT OF DIPEPTIDE COMPOUND, PREPARATION METHOD THEREFOR, AND USE THEREOF**

(30) Priority: 16.02.2023 CN 202310121070
(71) Applicant: Zhejiang Huahai Pharmaceutical Co., Ltd., Taizhou, Zhejiang 317024 (CN)
(72) Inventor: LI, Min, Shanghai 201203 (CN); HUANG, Yu, Shanghai 201203 (CN); SUN, Wen, Zhejiang 317024 (CN); WANG, Lulu, Shanghai 201203 (CN); JIN, Jianyang, Shanghai 201203 (CN)
(74) Representative: Ostertag & Partner Patentanwälte mbB
(86) International application number: PCT/CN2024/076192
(87) International publication number: WO 2024/169765

(57) **Abstract**

The application relates to a novel salt of compound (II) and a crystalline form thereof, and to a preparation method for the salt and the crystalline form thereof. Additionally, the application provides a pharmaceutical composition comprising a hydrochloride of compound (II), and an application of the salt in the preparation of a drug used for the prevention and treatment of thrombin-mediated and/or thrombin-related diseases.

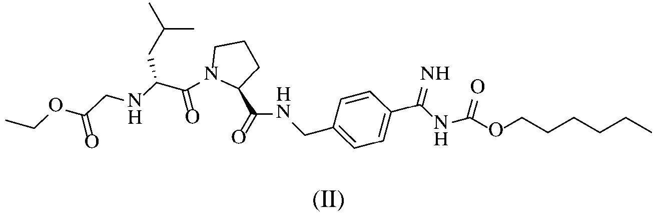

## Description

The present application claims the priority of Chinese Patent Application No. 202310121070.1 filed on February 16, 2023, which is incorporated herein by reference in its entirety.

### Technical Field

The present application relates to the field of pharmaceutical chemical engineering, in particular to a salt of a dipeptide compound, a preparation method and use thereof.

### Background

U.S. Patent US17289379 discloses a compound represented by formula II (in short compound II), which can be used to mediate the activity of trypsin-like serine proteases, and can be used as an anticoagulant and as an agent for modulating or inhibiting the activity of trypsin-like serine proteases, and thereby treating thromboembolic diseases and other related cardiovascular diseases.

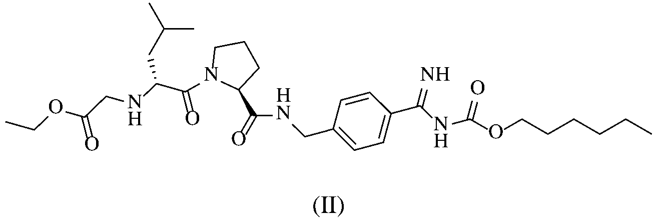

Blood has both a coagulation system and an anticoagulation system (fibrinolytic system). Physiologically, there is a continuous and limited activation of blood coagulation factors in the blood, resulting in the production of thrombin and the formation of trace amounts of fibrin. After formation, these fibrins are deposited in the intima of the cardiovascular system, and activation of the fibrinolytic system allows for the timely dissolution of small amounts of deposited fibrin. Thus, the coagulation system and the anticoagulation system (fibrinolytic system) ensure both the potential coagulability and the fluidity of the blood. When certain factors that trigger thrombosis occur, the above dynamic equilibrium is disturbed, thus triggering a coagulation reaction. The coagulation reaction primarily includes a primary coagulation process and a secondary coagulation process. The secondary coagulation process involves a series of biochemical reactions (the blood coagulation cascade) that includes the activation of inactive enzymes (or proenzymes) of blood coagulation factors to serine proteases (form factor X to factor Xa), which can sequentially activate subsequent blood coagulation factors (factor Xa activates factor II to form factor IIa), and ultimately converting the soluble plasma protein fibrinogen into insoluble plasma protein. Fibrin thrombin is an extracellular insulin-like serine protease that plays a key role in the blood coagulation cascade and is the final enzyme in the process. If the activity of thrombin can be inhibited, the formation of thrombus can be blocked. After oral administration, compound II is degraded *in vivo* by carboxylesterases (carboxylesterase-1/ carboxylesterase-2, CES-1/CES-2) through two intermediate states to the active metabolite, compound V. Compound V exerts its anticoagulant effect by selectively inhibiting thrombin.

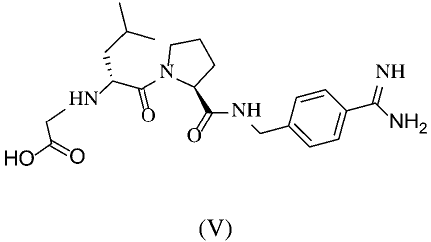

However, excessive inhibition of thrombin activity may cause a risk of bleeding, and therefore precise clinical control of the dosage of the anticoagulant is required.

### Summary of the Invention

In one aspect, the present application provides a crystalline form of a hydrochloride of a compound represented by formula II,

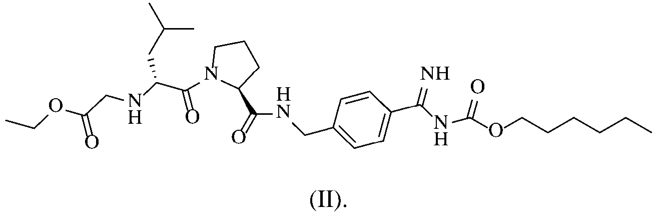

In some embodiments of the present application, the stoichiometric ratio of the compound of formula II and HCl is 1:1 or 1:2.

In another aspect, the present application provides a Form A of a monohydrochloride of the compound of formula II, wherein an X-ray diffraction pattern of the Form A has diffraction peaks at 2θ = 6.7 ± 0.2°, 16.2 ± 0.2°, 21.8 ± 0.2°, and 22.9 ± 0.2°;
typically has diffraction peaks at 2θ = 6.7 ± 0.2°, 16.2 ± 0.2°, 16.5 ± 0.2°, 20.7 ± 0.2°, 21.8 ± 0.2°, and 22.9 ± 0.2°; and
more typically has diffraction peaks at 2θ = 6.7 ± 0.2°, 7.8 ± 0.2°, 16.2 ± 0.2°, 16.5 ± 0.2°, 19.4 ± 0.2°, 20.7 ± 0.2°, 21.8 ± 0.2°, 22.9 ± 0.2°, 23.8 ± 0.2°, and 27.5 ± 0.2° as measured using Cu-Kα radiation.

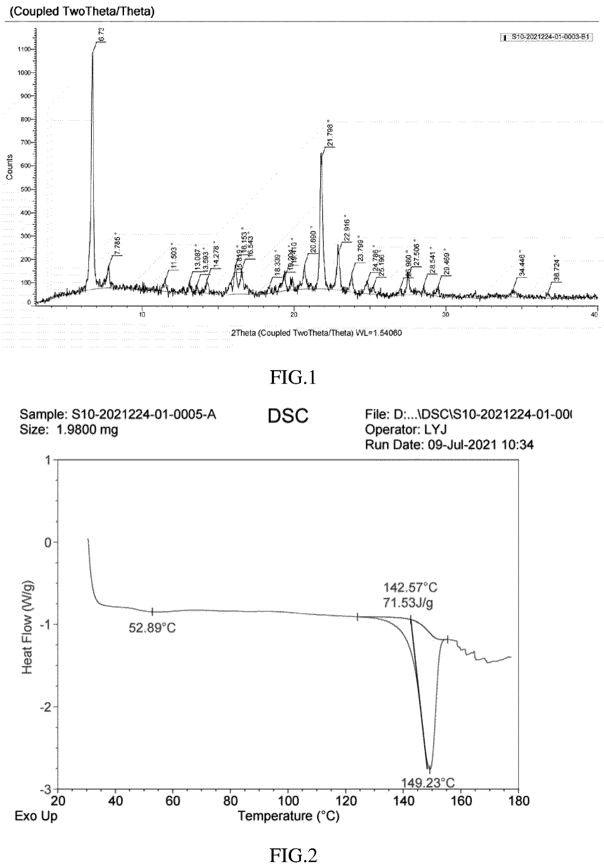

In some embodiments of the present application, the Form A of the monohydrochloride of the compound of formula II has an X-ray diffraction pattern as measured using Cu-Kα radiation that is substantially identical to that shown in FIG. 1.

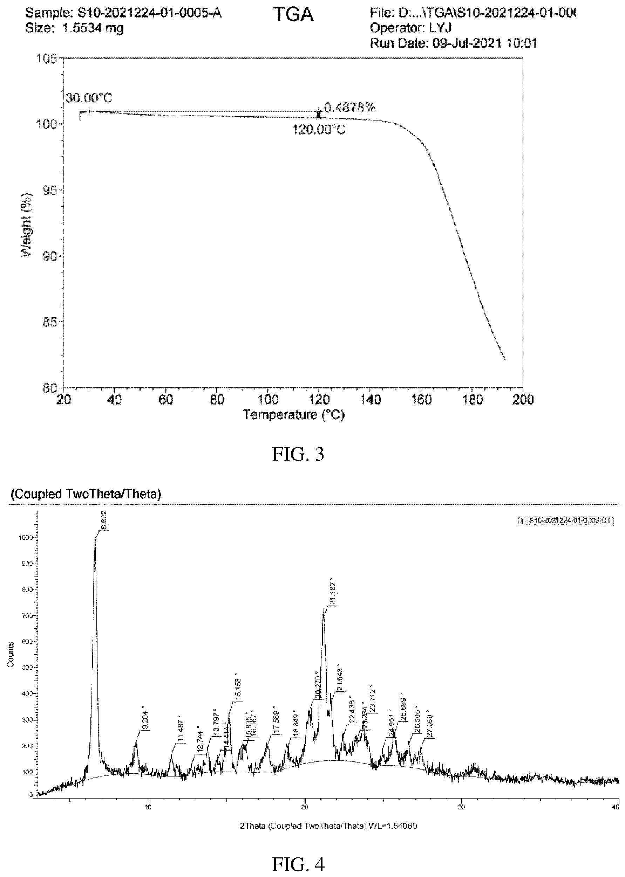

In some embodiments of the present application, the Form A of the monohydrochloride of the compound of formula II has a DSC pattern substantially identical to that shown in FIG. 2 or a TGA pattern substantially identical to that shown in FIG. 3.

In another aspect, the present application provides a Form B of a monohydrochloride of the compound of formula II, wherein an X-ray diffraction (XRD) pattern of the Form B has diffraction peaks at 2θ = 6.6 ± 0.2°, 15.2 ± 0.2°, 21.2 ± 0.2°, and 21.6 ± 0.2°; typically has diffraction peaks at 2θ = 6.6 ± 0.2°, 15.2 ± 0.2°, 20.3 ± 0.2°, 21.2 ± 0.2°, 21.6 ± 0.2°, and 23.7 ± 0.2°; and more typically has diffraction peaks at 2θ = 6.6 ± 0.2°, 9.2 ± 0.2°, 15.2 ± 0.2°, 17.6 + 0.2°, 20.3 ± 0.2°, 21.2 ± 0.2°, 21.6 ± 0.2°, 22.4 ± 0.2°, 23.7 ± 0.2°, and 25.7 ± 0.2° as measured using with Cu-Kα radiation.

In some embodiments of the present application, the Form B of the monohydrochloride of the compound of formula II has an X-ray diffraction pattern as measured using Cu-Kα radiation substantially identical to that shown in FIG. 4.

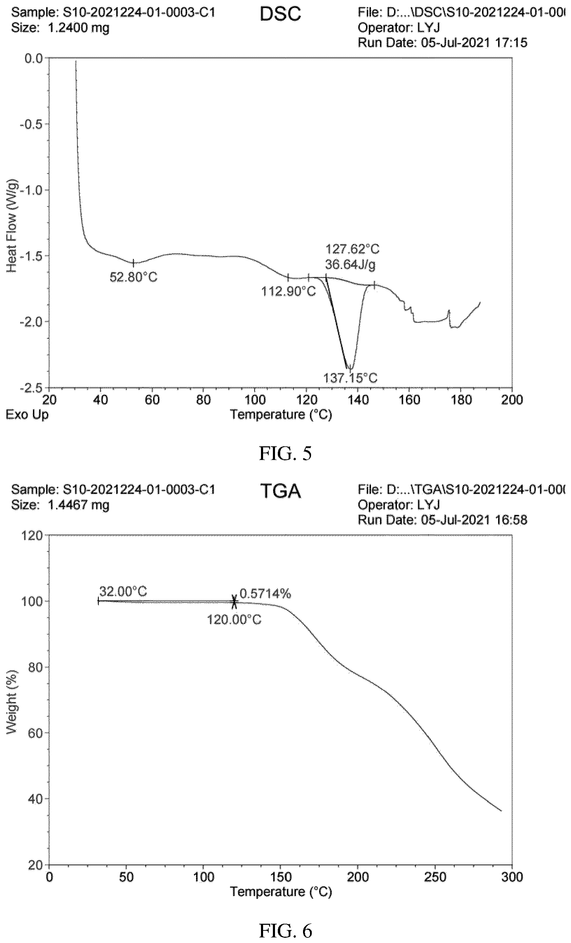

In some embodiments of the present application, the Form B of the monohydrochloride of the compound of formula II has a DSC pattern substantially identical to that shown in FIG. 5 or a TGA pattern substantially identical to that shown in FIG. 6.

The present application also provides a dihydrochloride of the compound of formula II.

In another aspect, the present application provides a Form I of a dihydrochloride of the compound of formula II, wherein an X-ray diffraction (XRD) pattern of the Form I has diffraction peaks at 2θ = 6.0 ± 0.2°, 17.1 ± 0.2°, 19.9 ± 0.2°, and 23.3 ± 0.2°; typically has diffraction peaks at 2θ = 6.0 ± 0.2°, 17.1 ± 0.2°, 19.9 ± 0.2°, 23.3 ± 0.2°, 25.4 ± 0.2°, and 25.7 ± 0.2°; and more typically has diffraction peaks at 2θ = 6.0 ± 0.2°, 13.1 ± 0.2°, 17.1 ± 0.2°, 17.5 ± 0.2°, 19.9 ± 0.2°, 20.2 ± 0.2°, 22.0 ± 0.2°, 23.3 ± 0.2°, 25.4 ± 0.2°, and 25.7 ± 0.2° as measured using Cu-Kα radiation.

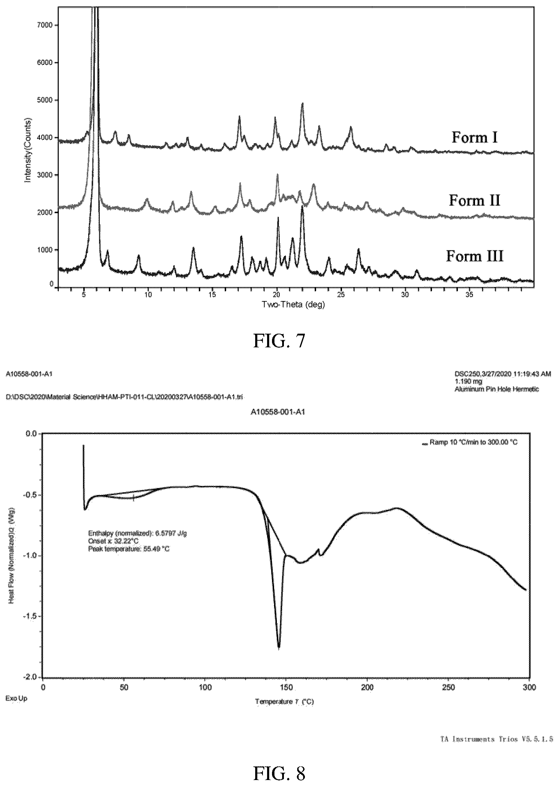

In some embodiments of the present application, the Form I of the dihydrochloride of the compound of formula II has an X-ray diffraction pattern as measured using Cu-Kα radiation that is substantially identical to that shown in the Form I of FIG. 7.

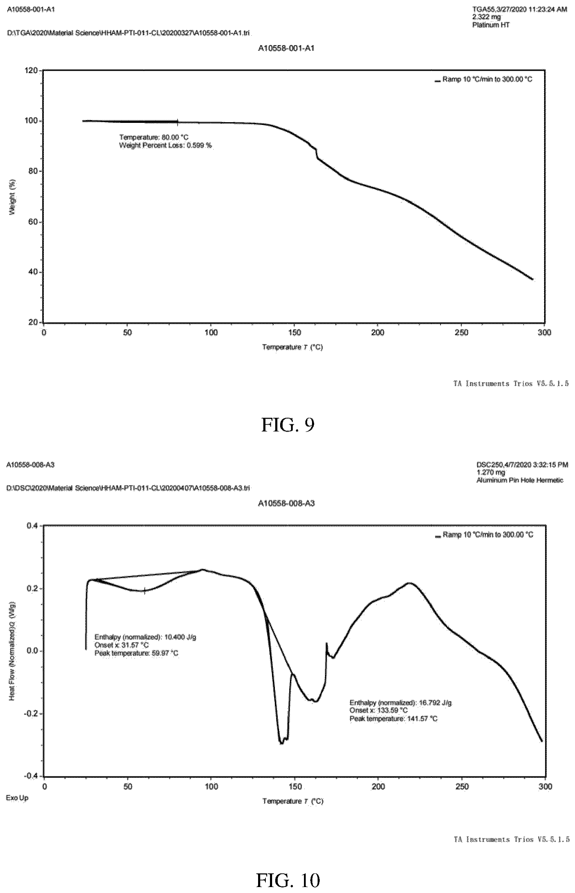

In some embodiments of the present application, the Form I of the dihydrochloride of the compound of formula II has a DSC pattern substantially identical to that shown in FIG. 8 or a TGA pattern substantially identical to that shown in FIG. 9.

In another aspect, the present application also provides a Form II of a dihydrochloride of the compound of formula II, wherein an X-ray diffraction (XRD) pattern of the Form II has diffraction peaks at 2θ = 6.0 ± 0.2°, 17.1 ± 0.2°, 20.0 ± 0.2°, and 22.8 ± 0.2°; typically has diffraction peaks at 2θ = 6.0 ± 0.2°, 13.4 ± 0.2°, 17.1 ± 0.2°, 20.0 ± 0.2°, 21.2 ± 0.2°, and 22.8 ± 0.2°; and more typically has diffraction peaks at 2θ = 6.0 ± 0.2°, 9.9 ± 0.2°, 13.4 ± 0.2°, 17.1 ± 0.2°, 20.0 ± 0.2°, 20.5 ± 0.2°, 21.2 ± 0.2°, 22.8 ± 0.2°, 26.9 ± 0.2°, and 30.2±0.2° as measured using Cu-Kα radiation.

In some embodiments of the present application, the Form II of the dihydrochloride of the compound of formula II has an X-ray diffraction pattern as measured using Cu-Kα radiation that is substantially identical to that shown in the Form II of FIG. 7.

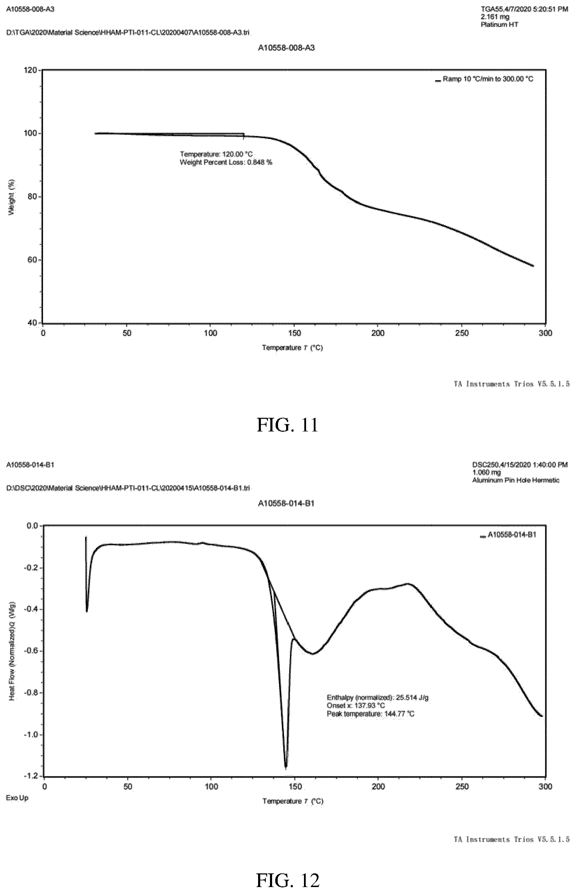

In some embodiments of the present application, the Form II of the dihydrochloride of the compound of formula II has a DSC pattern substantially identical to that shown in FIG. 10 or a TGA pattern substantially identical to that shown in FIG. 11.

The present application also provides a Form III of a dihydrochloride of the compound of formula II, wherein an X-ray diffraction (XRD) pattern of the Form II has diffraction peaks at 2θ = 6.0 ± 0.2°, 17.2 ± 0.2°, 20.1 ± 0.2°, and 22.0 ± 0.2°; typically has diffraction peaks at 2θ = 6.0 ± 0.2°, 13.5 ± 0.2°, 17.2 ± 0.2°, 20.1 ± 0.2°, 21.2 ± 0.2°, and 22.0 ± 0.2°; and more typically has diffraction peaks at 2θ = 6.0 ± 0.2°, 9.3 ± 0.2°, 13.5 ± 0.2°, 17.2 ± 0.2°, 18.7 ± 0.2°, 20.1 ± 0.2°, 22.0 ± 0.2°, 21.2 ± 0.2°, 24.0 ± 0.2° and 26.4 ± 0.2° as measured using Cu-Kα radiation.

In some embodiments of the present application, the Form III of the dihydrochloride of the compound of formula II has an X-ray diffraction pattern as measured using Cu-Kα radiation that is substantially identical to that shown in the Form III of FIG. 7.

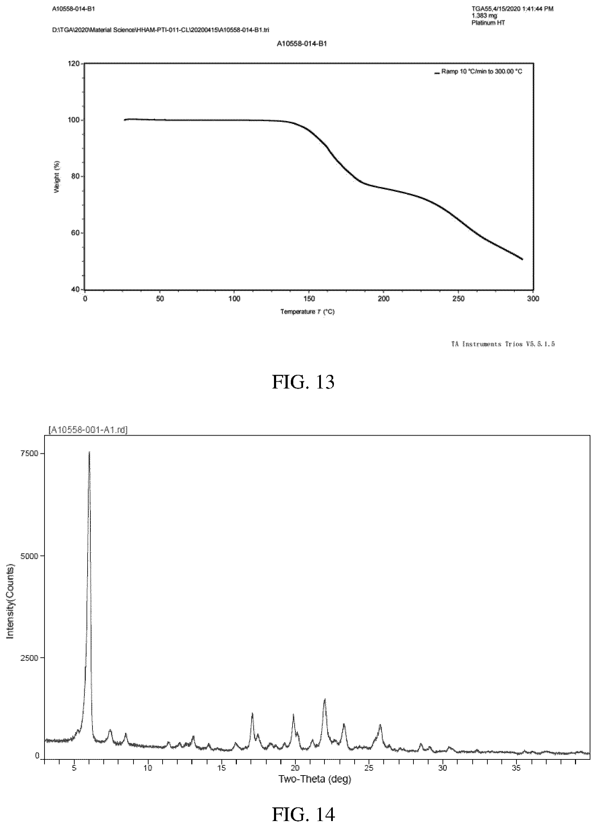

In some embodiments of the present application, the Form III of the dihydrochloride of the compound of formula II has a DSC pattern substantially identical to that shown in FIG. 12 or a TGA pattern substantially identical to that shown in FIG. 13.

The present application also provides a preparation method for the Form A of the monohydrochloride of the compound of formula II, comprising following steps:
(a) dissolving a free alkali of the compound of formula II in a mixed solvent of methyl ethyl ketone/n-heptane, and stirring to obtain a clarified solution; and
(b) adding slowly a solution of HCl in ethyl acetate to the clarified solution, stirring, filtering, and drying.

In some embodiments of the present application, in the preparation method for the Form A, the volume ratio of methyl ethyl ketone/n-heptane in step (a) may be 1:1; and the acid-base feeding ratio of HCl and the free alkali of the compound of formula II in step (b) is 1:1 to 2:1, such as 1.3:1, 1.4:1, 1.5:1, 1.6:1, 1.7:1, 1.8:1, 1.9:1, and 2:1, preferably 1.1:1 to 1.5:1.

In some embodiments of the present application, in the preparation method for the Form A, dissolving the free alkali of the compound of formula II at room temperature in step (a), and stirring at room temperature for 24 h in step (b).

In another aspect, the present application provides a preparation method for the Form B of the monohydrochloride of the compound of formula II, comprising following steps:
(a) dissolving a free alkali of the compound of formula II in ethyl acetate, and stirring to obtain a clarified solution; and
(b) adding slowly a solution of HCl in ethyl acetate to the clarified solution, stirring, filtering, and drying.

In some embodiments of the present application, in the preparation method for the Form B, the acid-base feeding ratio of the HCl and the free alkali of the compound of formula II in step (b) is 1:1 to 2:1, such as 1.1:1, 1.3:1, 1.4:1, 1.5:1, 1.6:1, 1.7:1, 1.8:1, 1.9:1, and 2:1, preferably 1.1:1 to 1.5:1.

In some embodiments herein, in the preparation method for the Form B, dissolving the free alkali of the compound of formula II at room temperature in step (a), and stirring at room temperature for 24 h in step (b).

In another aspect, the present application provides a preparation method for the Form I of the dihydrochloride of the compound of formula II, comprising following steps:
(a) dissolving a free alkali of the compound of formula II in ethyl acetate, and stirring to obtain a clarified solution; and
(b) adding slowly a solution of HCl in ethyl acetate to the clarified solution, stirring, filtering, and drying.

In some embodiments herein, in the preparation method for the Form I, the acid-base feeding ratio of the HCl and the free alkali of the compound of formula II in step (b) is 2:1 to 5:1, such as 2.5:1, 3:1, 3.5:1, 4:1, and 4.2:1, preferably 2.5:1 to 4:1.

In some embodiments herein, in the preparation method for the Form I, dissolving the free alkali of the compound of formula II at room temperature in step (a), and stirring at low temperature for 5 h in step (b).

In another aspect, the present application provides a preparation method for the Form II of the dihydrochloride of the compound of formula II, comprising following steps: suspending the Form I of the dihydrochloride of the compound of formula II in a ketone solvent, slurrying, and filtering and drying.

The ketone solvent described for the Form II of the compound of formula II is selected from the group consisting of acetone, butanone, methyl ethyl ketone, and combinations thereof.

In some embodiments of the present application, in the preparation method for the Form II, the slurrying is performed at a temperature from room temperature to 50 °C for 24 h.

In another aspect, the present application provides a preparation method for the Form III of the dihydrochloride of the compound of formula II, comprising following steps: suspending the Form I of the dihydrochloride of the compound of formula II in methyl tert-butyl ether, slurrying, and filtering and drying.

In some embodiments of the present application, in the preparation method for the Form III, the slurrying is performed at a temperature from room temperature to 50 °C for 24 h.

The present application also provides a pharmaceutical composition comprising the crystalline form of the hydrochloride of the compound of formula II, or the monohydrochloride and/or the dihydrochloride of the compound of formula II, together with one or more pharmaceutically acceptable inert pharmaceutical adjuvant(s).

The present application also provides a pharmaceutical composition comprising 95% or more (e.g., 95%, 96%, 97%, 98%, 99%, or any value or range therebetween) of the dihydrochloride of the compound of formula II.

The present application also provides a crystal composition comprising 80% or more (e.g. 80%, 90%, 95%, or any value or range therebetween) of the Form I of the hydrochloride of the compound of formula II.

In some embodiments of the present application, the pharmaceutical composition of the present application is formulated for oral administration.

In another aspect, the present application provides use of the crystalline form of the hydrochloride of the compound of formula II, the monohydrochloride of the compound of formula II, the dihydrochloride of the compound of formula II, the pharmaceutical composition, or the crystal composition, as defined above, in the manufacture of a medicament for the prevention and treatment of thrombin-mediated and thrombin-related diseases.

In another aspect, the present application provides use of the crystalline form of the hydrochloride of the compound of formula II, the monohydrochloride of the compound of formula II, the dihydrochloride of the compound of formula II, the pharmaceutical composition, or the crystal composition, as defined above, in the manufacture of a medicament for the treatment of venous and/or arterial thrombotic diseases.

### Brief Description of the Drawings

FIG. 1 shows an XRD pattern of the Form A of the monohydrochloride of the compound of formula II.
FIG. 2 shows a DSC pattern of the Form A of the monohydrochloride of the compound of formula II.
FIG. 3 shows a TGA pattern of the Form A of the monohydrochloride of the compound of formula II.
FIG. 4 shows an XRD pattern of the Form B of the monohydrochloride of the compound of formula II.
FIG. 5 shows a DSC pattern of the Form B of the monohydrochloride of the compound of formula II.
FIG. 6 shows a TGA pattern of the Form B of the monohydrochloride of the compound of formula II.
FIG. 7 shows an XRD pattern of the Form I, the Form II, and the Form III of the dihydrochloride of the compound of formula II.
FIG. 8 shows a DSC pattern of the Form I of the dihydrochloride of the compound of formula II.
FIG. 9 shows a TGA pattern of the Form I of the dihydrochloride of the compound of formula II.
FIG. 10 shows a DSC pattern of the Form II of the dihydrochloride of the compound of formula II.
FIG. 11 shows a TGA pattern of the Form II of the dihydrochloride of the compound of formula II.
FIG. 12 shows a DSC pattern of the Form III of the dihydrochloride of the compound of formula II.
FIG. 13 shows a TGA pattern of the Form III of the dihydrochloride of the compound of formula II.
FIG. 14 shows an XRD pattern of the Form I of the dihydrochloride of the compound of formula II.

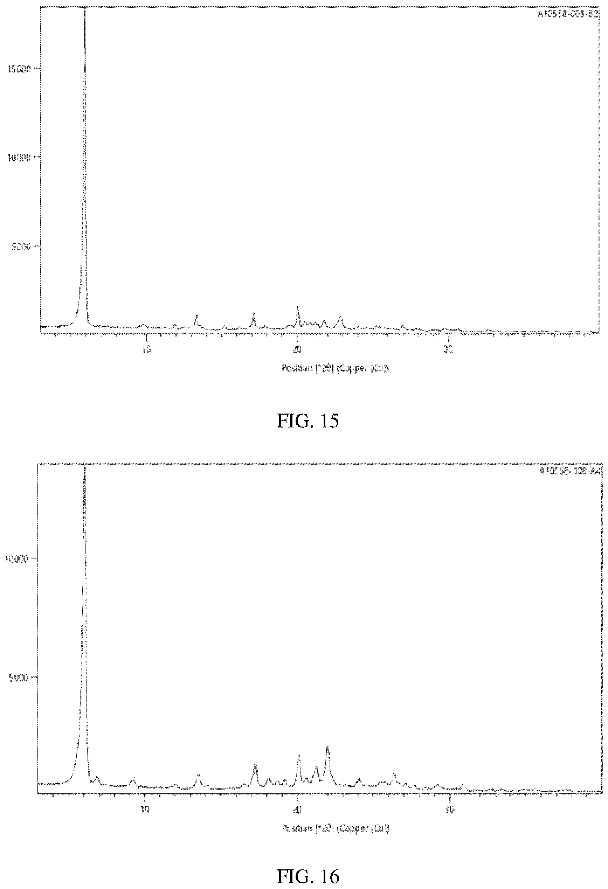

FIG. 15 shows an XRD pattern of the Form II of the dihydrochloride of the compound of formula II.
FIG. 16 shows an XRD pattern of the Form III of the dihydrochloride of the compound of formula II.

### Detailed Description

In the following description, certain specific details are included to provide a thorough understanding of various disclosed embodiments. However, those skilled in the art will recognize that embodiments may be practiced without one or more of these specific details, but with other methods, components, materials, etc.

As used herein, "in some embodiments" means that specific reference elements, structures or features related to that embodiment are included in at least one embodiment. Therefore, the phrase "in some embodiments" which appears in different places throughout the specification are not necessarily all referring to the same embodiment. Furthermore, specific elements, structures or features may be combined in one or more embodiments in any suitable manner.

In one aspect, the present application provides a hydrochloride of a compound of formula II, comprising a monohydrochloride, a dihydrochloride, and the like. Specifically, the monohydrochloride of the compound of formula II has a stoichiometric ratio of the compound of formula II: HCl of 1:1, and can exist in an anhydrous crystalline form, a hydrate form, a solvate form, and the like. The dihydrochloride of the compound of formula II has a stoichiometric ratio of the compound of formula II: HCl of 1:2, and can exist in an anhydrous crystalline form, a hydrate form, a solvate form, and the like.

In the present application, the hydrochloride of the compound of formula II is a crystalline salt, and when compared to the free alkali, the hydrochloride of the compound of formula II has improved pharmacological properties, excellent preservation properties (e.g., stability), and is easily formulated into pharmaceutical composition such as tablets or capsules, etc. For example, the free alkali of the compound of formula II is an oil, whereas the monohydrochloride or dihydrochloride of the compound of formula II is a crystalline compound; the free alkali of the compound of formula II is insoluble in water, whereas the monohydrochloride or dihydrochloride of the compound of formula II has an excellent aqueous solubility and an intrinsic dissolution rate, which improves bioavailability, and these properties make it particularly suitable for use in pharmaceutical applications. In addition, after conversion of the free form to the monohydrochloride or dihydrochloride form, certain process impurities which are difficult to remove in the free form are surprisingly present to a much lesser extent, and the monohydrochloride or dihydrochloride is easier to separate than the free alkali, so that the formation of the salt may provide a purification method.

Any suitable method known in the art for separating the crystalline hydrochloride may be used. Suitably, the monohydrochloride or dihydrochloride of the compound of formula II is obtained by filtration separation and collection. Preferably, the monohydrochloride or dihydrochloride of the compound of formula II is obtained by drying under vacuum.

Each crystalline form of the hydrochloride described herein is in a substantially pure form.

As used herein, the term "substantially pure" means at least 95% purity, preferably 98% purity, wherein 95% purity means no more than 5%, and 98% purity means no more than 2% of any other form of the compound of formula II (other crystalline form, amorphous form, etc.) is present.

In another aspect, the present application provides a Form A of the monohydrochloride of the compound of formula II.

In some embodiments of the present application, wherein an X-ray diffraction pattern of the Form A of the monohydrochloride of the compound of formula II has diffraction peaks at 2θ = 6.7 ± 0.2°, 16.2 ± 0.2°, 21.8 ± 0.2°, and 22.9 ± 0.2°; typically has diffraction peaks at 2θ = 6.7 ± 0.2°, 16.2 ± 0.2°, 16.5 ± 0.2°, 20.7 ± 0.2°, 21.8 ± 0.2°, and 22.9 ± 0.2°; and more typically has diffraction peaks at 2θ = 6.7 ± 0.2°, 7.8 ± 0.2°, 16.2 ± 0.2°, 16.5 ± 0.2°, 19.4 ± 0.2°, 20.7 ± 0.2°, 21.8 ± 0.2°, 22.9 ± 0.2°, 23.8 ± 0.2°, and 27.5 ± 0.2° as measured using Cu-Kα radiation.

In some embodiments of the present application, the Form A of the monohydrochloride of the compound of formula II has an X-ray diffraction pattern as measured using Cu-Kα radiation that is substantially identical to that shown in FIG. 1.

In some embodiments of the present application, the Form A of the monohydrochloride of the compound of formula II has characterization data that is substantially identical to that as shown in Table 1.

**Table 1 - Characterization data of the Form A of the monohydrochloride**

| **Crystalline Form** | **TGA weight loss (wt%)** | **DSC Onset/Peak (°C)** | **Moisture adsorption content (wt%)** | **Stability at room temperature (10 days)** |
|---|---|---|---|---|
| Form A of the monohydrochloride | 0.5 | 52.9, 149.2 | 0.5 (Slightly hygroscopic) | White powder, crystalline form remains unchanged (10 days) |

In some embodiments of the present application, the Form A of the monohydrochloride of the compound of formula II has a DSC pattern substantially identical to that shown in FIG. 2 or a TGA pattern substantially identical to that shown in FIG. 3.

The present application also provides a Form B of the monohydrochloride of the compound of formula II.

In some embodiments of the present application, wherein an X-ray diffraction pattern of the Form B of the monohydrochloride of the compound of formula II has diffraction peaks at 2θ = 6.6 ± 0.2°, 15.2 ± 0.2°, 21.2 ± 0.2°, and 21.6 ± 0.2°; typically has diffraction peaks at 2θ = 6.6 ± 0.2°, 15.2 ± 0.2°, 20.3 ± 0.2°, 21.2 ± 0.2°, 21.6 ± 0.2°, and 23.7 ± 0.2°; and more typically has diffraction peaks at 2θ = 6.6 ± 0.2°, 9.2 ± 0.2°, 15.2 ± 0.2°, 17.6 ± 0.2°, 20.3 ± 0.2°, 21.2 ± 0.2° , 21.6 ± 0.2°, 22.4 ± 0.2°, 23.7 ± 0.2°, and 25.7 ± 0.2° as measured using Cu-Kα radiation.

In some embodiments of the present application, the Form B of the monohydrochloride of the compound of formula II has an X-ray diffraction pattern as measured using Cu-Kα radiation substantially identical to that shown in FIG. 4.

In some embodiments of the present application, the Form B of the monohydrochloride of the compound of formula II has characterization data that is substantially identical to that as shown in Table 2.

**Table 2 - Characterization data of the Form B of the monohydrochloride**

| **Crystalline Form** | **TGA weight loss (wt%)** | **DSC Onset/Peak (°C)** | **Moisture adsorption content (wt%)** | **Stability at room temperature (10 days)** |
|---|---|---|---|---|
| Form B of the monohydrochloride | 1.0 | 52.8, 137.2 | 3.0 (Hygroscopic) | White powder, crystalline form remains unchanged (10 days) |

In some embodiments of the present application, the Form B of the monohydrochloride of the compound of formula II has a DSC pattern substantially identical to that shown in FIG. 5 or a TGA pattern substantially identical to that shown in FIG. 6.

The present application also provides the dihydrochloride of the compound of formula II.

The present application also provides a Form I of the dihydrochloride of the compound of formula II.

In some embodiments of the present application, an X-ray diffraction (XRD) pattern of the Form I of the dihydrochloride of the compound of formula II has diffraction peaks at 2θ = 6.0 ± 0.2°, 17.1 ± 0.2°, 19.9 ± 0.2°, and 23.3 ± 0.2°; typically has diffraction peaks at 2θ = 6.0 ± 0.2°, 17.1 ± 0.2°, 19.9 ± 0.2°, 23.3 ± 0.2°, 25.4 ± 0.2°, and 25.7 ± 0.2°; and more typically has diffraction peaks at 2θ = 6.0 ± 0.2°, 13.1 ± 0.2°, 17.1 ± 0.2°, 17.5 ± 0.2°, 19.9 ± 0.2°, 20.2 ± 0.2°, 22.0 ± 0.2°, 23.3 ± 0.2°, 25.4 ± 0.2°, and 25.7 ± 0.2° as measured using Cu-Kα radiation.

In some embodiments of the present application, the Form I of the dihydrochloride of the compound of formula II has an X-ray diffraction pattern as measured using Cu-Kα radiation that is substantially identical to that shown in the Form I of FIG. 7.

In some embodiments of the present application, the Form I of the dihydrochloride of the compound of formula II has characterization data that is substantially identical to that as shown in Table 3.

**Table 3 Summary of DSC and TGA characteristics of the Form I of the dihydrochloride**

| **Crystalline Form** | **DSC Onset/Peak (°C), ΔH (J/g)** | **TGA weight loss (wt%)** |
|---|---|---|
| Form I of the dihydrochloride, hydrate | 139/145, 33 | About 0.6 |

In some embodiments of the present application, the Form I of the dihydrochloride of the compound of formula II has a DSC pattern substantially identical to that shown in FIG. 8 or a TGA pattern substantially identical to that shown in FIG. 9.

In some embodiments of the present application, the DSC pattern of the Form I of the dihydrochloride of the compound of formula II shows an endothermic peak at 139 °C and a decomposition peak at 145 °C; and the TGA pattern shows a weight loss of about 0.6% by 80 °C.

The DVS results for the Form I show that the water absorption of the Form I is 28.8%/49.9% at 80% RH/90% humidity. The sample undergoes deliquescence at high humidity and the sample is converted to amorphous form after DVS test.

The physicochemical properties of the Form I are as follows:

| Characteristics | Off-white to white solid |
|---|---|
| Hygroscopicity | Hygroscopic |
| pKa | p*K*a1 = 4.94, p*K*a2 = 5.54, p*K*a3 = 10.61 |
| Partition coefficient | Log P > 3.38, Log D_{7.4} = 2.06 |
| Solubility | Highly soluble in buffer solutions with different pH values (1.2, 2.0, 3.0, 4.0, 5.0, 6.0, 7.0, 8.0, 9.0, 10.0, 11.0, 12.0 and 13.0), readily soluble in acetonitrile and dichloromethane, and almost insoluble or insoluble in n-heptane and *N, N*-diisopropyl ethylamine. |

The solubility of the Form I was determined as follows:

| **No.** | **Solvent** | **Solubility (mg/mL)** |
|---|---|---|
| 1 | EtOH | >250 |
| 2 | IPA | ~167 |
| 3 | NBA | ~167 |
| 4 | MEK | ~1.6 |
| 5 | EA | <0.3 |
| 6 | IPAc | -0.5 |
| 7 | Hept | <0.3 |
| 8 | Diox | ~125 |
| 9 | THF | ~11.1 |
| 10 | Tol | <0.3 |
| 11 | ACN | >250 |
| 12 | Water | >250 |
| 13 | MTBE | <0.3 |
| 14 | DMSO | >250 |
| 15 | AC | >250 |

The accelerated stability test data of the Form I at T = 25 ± 2 °C, 60 ± 5% humidity are as follows:

| **Detection period** | **Moisture** | **Hydrolyzed impurities** | **Other individual impurities** | **Total impurities** | **Content (HPLC)** |
|---|---|---|---|---|---|
| **0 month** | 0.4% | 0.19% | <RL(0.05%) | 0.42% | 99.6% |
| **1 month** | 0.5% | 0.27% | <RL(0.05%) | 0.54% | 99.3% |
| **2 months** | 0.3% | 0.28% | <RL(0.05%) | 0.56% | 100.3% |
| **3 months** | 0.4% | 0.45% | <RL(0.05%) | 0.72% | 100.3% |
| **6 months** | 0.3% | 0.51% | <RL(0.05%) | 1.02% | 99.6% |

The present application also provides a Form II of the dihydrochloride of the compound of formula II.

In some embodiments of the present application, an X-ray diffraction (XRD) pattern of the Form II of the dihydrochloride of the compound of formula II has diffraction peaks at 2θ = 6.0 ± 0.2°, 17.1 ± 0.2°, 20.0 ± 0.2°, and 22.8 ± 0.2°; typically has diffraction peaks at 2θ = 6.0 ± 0.2°, 13.4 ± 0.2°, 17.1 ± 0.2°, 20.0 ± 0.2°, 21.2 ± 0.2°, and 22.8 ± 0.2°; and more typically has diffraction peaks at 2θ = 6.0 ± 0.2°, 9.9 ± 0.2°, 13.4 ± 0.2°, 17.1 ± 0.2°, 20.0 ± 0.2°, 20.5 ± 0.2°, 21.2 ± 0.2°, 22.8 ± 0.2°, 26.9 ± 0.2°, and 30.2 ± 0.2° as measured using Cu-Kα radiation.

In some embodiments of the present application, the Form II of the dihydrochloride of the compound of formula II has an X-ray diffraction pattern as measured using Cu-Kα radiation that is substantially identical to that shown in the Form II of FIG. 7.

In some embodiments of the present application, the Form II of the dihydrochloride of the compound of formula II has characterization data that is substantially identical to that as shown in Table 4.

**Table 4 Summary of DSC and TGA characteristics of the Form II of the dihydrochloride**

| **Crystalline Form** | **DSC Onset/Peak (°C), ΔH (J/g)** | **TGA weight loss (wt%)** |
|---|---|---|
| Form II of the dihydrochloride, anhydrous | 134/142, 17 | About 0.8 |

In some embodiments of the present application, the Form II of the dihydrochloride of the compound of formula II has a DSC pattern substantially identical to that shown in FIG. 10 or a TGA pattern substantially identical to that shown in FIG. 11.

In some embodiments of the present application, the DSC pattern of the Form II of the dihydrochloride of the compound of formula II shows an endothermic peak at 134 °C and a decomposition peak at 142 °C; and the TGA pattern shows a weight loss of about 0.8% by 120 °C.

There is a broad endothermic peak before 100 °C in the DSC curve for the Form II. When heating to 110 °C, the Form in DSC does not change. In some embodiments of the present invention, about 0.2% acetone residue is present when the Form II is obtained by slurrying in acetone.

The present application also provides a Form III of the dihydrochloride of the compound of formula II.

In some embodiments of the present application, an X-ray diffraction (XRD) pattern of the Form III of the dihydrochloride of the compound of formula II has diffraction peaks at 2θ = 6.0 ± 0.2°, 17.2 ± 0.2°, 20.1 ± 0.2°, and 22.0 ± 0.2°; typically has diffraction peaks at 2θ = 6.0 ± 0.2°, 13.5 ± 0.2°, 17.2 ± 0.2°, 20.1 ± 0.2°, 21.2 ± 0.2°, and 22.0 ± 0.2°; and more typically has diffraction peaks at 2θ = 6.0 ± 0.2°, 9.3 ± 0.2°, 13.5 ± 0.2°, 17.2 ± 0.2°, 18.7 ± 0.2°, 20.1 ± 0.2°, 22.0 ± 0.2°, 21.2 ± 0.2°, 24.0 ± 0.2°, and 26.4 ± 0.2° as measured using Cu-Kα radiation.

In some embodiments of the present application, the Form III of the dihydrochloride of the compound of formula II has an X-ray diffraction pattern as measured using Cu-Kα radiation that is substantially identical to that shown in the Form III of FIG. 7.

In some embodiments of the present application, the Form III of the dihydrochloride of the compound of formula II has characterization data that is substantially identical to that as shown in Table 5.

**Table 5 Summary of DSC and TGA characteristics of the Form III of the dihydrochloride**

| **Crystalline Form** | **DSC Onset/Peak (°C), ΔH (J/g)** | **TGA weight loss (wt%)** |
|---|---|---|
| Form III of the dihydrochloride, anhydrous | 138/145, 26 | About 0 |

In some embodiments of the present application, the Form III of the dihydrochloride of the compound of formula II has a DSC pattern substantially identical to that shown in FIG. 12 or a TGA pattern substantially identical to that shown in FIG. 13.

In some embodiments of the present application, the Form III of the dihydrochloride of the compound of formula II has a DSC pattern with an endothermic peak at 138 °C and a decomposition peak at 145 °C; and the TGA pattern shows no significant weight loss until 120 °C. The DVS results show that the water absorption at 80% RH/90% humidity is 28.8/50.0%.

The Form III is converted to Form I after mechanical milling for 2 minutes. The Form III is stored at 60 °C/sealed condition for 7 days and the purity decreased from 95.81% to 89.35% and is converted to Form I.

In another aspect, the present application provides a preparation method for each of the crystalline forms of the hydrochlorides of the compound of formula II, comprising mixing a free form of the compound of formula II in a suitable organic solvent with hydrogen chloride, and then filtering and drying after the formation of salt is completed. The method may further comprise stirring a slurry of the hydrochloride of the specific compound of formula II in a suitable organic solvent, and filtering and drying after the crystal transformation is completed. The solvent may be ethyl acetate, isopropyl acetate, methyl ethyl ketone, methyl tert-butyl ether, toluene, cyclohexane, isopropanol, acetonitrile, n-heptane, or a mixture of each solvent.

In some embodiments of the present application, the preparation method for the Form A of the monohydrochloride of the compound of formula II comprises following steps:
(a) dissolving a free alkali of the compound of formula II in a mixed solvent of methyl ethyl ketone/n-heptane and stirring to obtain a clarified solution; and
(b) adding slowly a solution of HCl in ethyl acetate to the clarified solution, stirring, filtering, and drying.

In some embodiments of the present application, in the preparation method for the Form A, the volume ratio of methyl ethyl ketone/n-heptane in step (a) may be 1:1; and the acid-base feeding ratio of the HCl and the free alkali of the compound of formula II in step (b) is 1:1 to 2:1, such as 1.3:1, 1.4:1, 1.5:1, 1.6:1, 1.7:1, 1.8:1, 1.9:1, and 2:1, preferably 1.1:1 to 1.5:1.

In some embodiments of the present application, in the preparation method for the Form A, dissolving the free alkali of the compound of formula II at room temperature in step (a), and stirring at room temperature for 24 h in step (b).

In some embodiments of the present application, the preparation method for the Form B of the monohydrochloride of the compound of formula II comprises following steps:
(a) dissolving a free alkali of the compound of formula II in ethyl acetate, and stirring to obtain a clarified solution; and
(b) adding slowly a solution of HCl in ethyl acetate to the clarified solution, stirring, filtering, and drying.

In some embodiments of the present application, in the preparation method for the Form B, the acid-base feeding ratio of the HCl and the free alkali of the compound of formula II in step (b) is 1:1 to 2:1, such as 1.1:1, 1.3:1, 1.4:1, 1.5:1, 1.6:1, 1.7:1, 1.8:1, 1.9:1, and 2:1, preferably 1.1:1 to 1.5:1.

In some embodiments of the present application, in the preparation method for the Form B, dissolving the free alkali of the compound of formula II at room temperature in step (a), and stirring at room temperature for 24 h in step (b).

During the preparation of the monohydrochloride, a portion of free alkali cannot be completely converted to the monohydrochloride when the acid-base feeding ratio is too low, and a mixture of monohydrochloride and dihydrochloride is produced when the acid-base feeding ratio is high. Therefore, it is necessary to strictly control the speed and amount of the addition of hydrochloric acid, which brings difficulties to industrial production and product quality control.

In some embodiments of the present application, the preparation method for the Form I of the dihydrochloride of the compound of formula II comprises following steps:
(a) dissolving a free alkali of the compound of formula II in ethyl acetate, and stirring to obtain a clarified solution; and
(b) adding slowly a solution of HCl in ethyl acetate to the clarified solution, stirring, filtering, and drying.

In some embodiments of the present application, in the preparation method for the Form I, the acid-base feeding ratio of the HCl and the free alkali of the compound of formula II in step (b) is 2:1 to 5:1, such as 2.5:1, 3:1, 3.5:1, 4:1, and 4.2:1, preferably 2.5:1 to 4:1. The excessive feeding amount of hydrochloric acid is conducive to the complete conversion of the free alkali to the dihydrochloride. In industrial production, the operation is simple, and the obtained Form I has few impurities and high purity.

In some embodiments of the present application, in the preparation method for the Form I, dissolving the free alkali of the compound of formula II at room temperature in step (a), and stirring at low temperature for 5 h in step (b).

In some embodiments of the present application, the preparation method for the Form II of the dihydrochloride of the compound of formula II comprises following steps: suspending the Form I of the dihydrochloride of the compound of formula II in a ketone solvent, slurrying, and filtering and drying.

In some embodiments of the present application, in the preparation method for the Form II, the ketone solvent is selected from the group consisting of acetone, butanone, methyl ethyl ketone, and combinations thereof, and the slurrying is performed at a temperature from room temperature to 50 °C for 24 h.

In some embodiments of the present application, the preparation method for the Form III of the dihydrochloride of the compound of formula II comprises following steps: suspending the Form I of the dihydrochloride of the compound of formula II in methyl tert-butyl ether, slurrying, and filtering and drying.

In some embodiments of the present application, in the preparation method for the Form III, the slurrying is performed at room temperature for 24 h.

In another aspect, the present application provides a pharmaceutical composition, comprising the hydrochloride of the compound of formula II as defined herein (such as the monohydrochloride of the compound of formula II or the dihydrochloride of the compound of formula II) and pharmaceutically acceptable inert pharmaceutical adjuvants.

In some embodiments of the present application, the pharmaceutical composition is formulated for oral administration such as tablets, capsules, powder granules, liquid formulations (e.g., oral liquids, suspensions, emulsions, etc.), or syrups, and the like, which may comprise pharmaceutically acceptable excipients such as lubricants, binders, disintegrating agents, fillers, dispersants, emulsifiers, stabilizers, and the like.

For example, injections may be made by mixing or dissolving the compound of the present application in physiological saline, which may include an appropriate amount of dilute acids or bases or buffer salts to adjust the pH to the most stable state, and may also include antioxidants or metal chelating agents. The solution is sterilized by filtration and filled in sterile ampoules under sterile conditions.

For example, tablets may be made by thoroughly mixing the compound of the present application with adjuvants (e.g., microcrystalline cellulose, sodium carboxymethyl starch, corn starch, magnesium stearate, talc, etc.), sieving, and tabletting on a tablet press. For example, hard capsule dosage forms may be made by sieving the compound of the invention, mixing it with adjuvants/excipients (e.g., dried starch, magnesium stearate, etc.), and filling the mixture in hard gelatin capsules by using appropriate equipment.

Suspensions may be prepared by sieving the compound of the present invention and mixing it with adjuvants (e.g., sodium carboxymethylcellulose and syrup) to form a homogeneous paste, and in some instances, pigments, benzoic acid, etc., may be diluted with a portion of purified water and added to the paste with stirring, and then adding enough water to produce the desired volume. Other methods adjuvants, and the like of preparing the pharmaceutical composition may be found in Remington's Pharmaceutical Sciences, 18th edition, Alfonso R. Gennaro (1990), published by Mack Publishing Company and revisions thereof.

The pharmaceutical compositions herein may be solid, semi-solid or liquid formulations made by conventional techniques known per se. These methods are, for example, mixing, dissolving, granulating, crushing, emulsifying, encapsulating, spray drying or freeze drying. The active ingredient in such compositions can range from 0.1% to 99.9% by weight of the formulation. The carriers, diluents or excipients used in the compositions are compatible with the active ingredient and are pharmaceutically acceptable.

In another aspect, the present application provides use of the hydrochloride of the compound of formula II for thrombin inhibition and for the prevention and treatment of thrombin-mediated and/or thrombin-related diseases.

As described in the examples section of the present application, representative hydrochlorides of the compound of formula II have significant antithrombotic effects in animal models. The present application relates to use of the compounds described herein, such as monohydrochloride of the compound of formula II or dihydrochloride of the compound of formula II, for the prevention and treatment of thrombotic diseases, in particular venous or arterial thromboembolism, such as lower extremity deep vein thromboembolism, reocclusion after bypass surgery or angioplasty, peripheral arterial disease occlusion, pulmonary embolism, disseminated intravascular coagulation, coronary artery thromboembolism, stroke, and occlusion of shunt or stent, etc.

The present application relates to use of the compounds described herein, such as monohydrochloride of the compound of formula II or dihydrochloride of the compound of formula II, for the prevention and treatment of stroke, pulmonary embolism, myocardial or cerebral infarction, atrial fibrillation and arrhythmia caused by thrombosis.

The present application relates to the use of the compounds described herein, such as monohydrochloride of the compound of formula II or dihydrochloride of the compound of formula II, for the prevention and treatment of atherosclerotic diseases such as coronary arterial disease, cerebral arterial disease and peripheral arterial disease.

The compounds described herein may also be used as anticoagulants in extracorporeal blood lines. In certain embodiments, the present invention provides a method of inhibiting coagulation in an extracorporeal or ex vivo blood line, and the method comprises adding an effective amount of the compound of the present invention to the extracorporeal or ex vivo blood line.

The compounds described herein (e.g., monohydrochloride of the compound of formula II or dihydrochloride of the compound of formula II) may also be used in combination with thrombolytic agents, e.g., to reduce the time of reperfusion and to prolong the time of reocclusion. In addition, the compounds described in the present invention may be used to prevent the re-formation of thrombi following microsurgery. The compounds described herein may also be useful in anticoagulant therapy for hemodialysis and in disseminated intravascular coagulation. The compounds described herein may also be used in the ex vivo preservation of blood, plasma and other blood products.

The compounds herein may be administered by the oral route. The specific administration dosage depends on the particular circumstances of the case, including the form of administration, the rate of administration, and the condition being treated, and the like. Typical oral daily doses that produce potency can range between about 0.01 mg/kg and about 1000 mg/kg (in terms of the free alkali of the compound of formula II). A single daily dose, or multiple doses of 2 to 4 times daily, may be administered. The dose and mode of administration may be adjusted based on the age and weight of the patient and the severity of the disease being treated.

In the present application, the compound of formula II was synthesized using the method described in US62751984.

In the present application, X-ray diffraction (XRD) was measured using the following method:
(1) X-ray powder diffraction data of the samples was collected under ambient conditions using a Bruker D2 model X-ray powder diffractometer with an X-ray emitter power of 300 W. The sample holder was free of background signals, with a step rate of 0.15 s/step, a total of 1,837 steps, a step size of 2θ = 0.02°, a voltage of 30 kV, and a current of 10 mA. The X-ray tube used a Cu target (Kα) with a Kα2/Kα1 intensity ratio of 0.50 (1.54439 Å/1.5406 Å).
(2) The analysis was performed using a PANalytical Empyrean X-ray powder diffractometer equipped with a PIXcellD detector. In the XRPD analysis, the samples were scanned at 2θ angles from 3° to 40° with a scanning step size of 0.013°, and the phototube voltage and phototube current were 45 KV and 40 mA, respectively.

In the present application, differential scanning calorimetry (DSC) was measured by the following method: the thermal data of the samples were collected by using a TA Discovery series of Differential Scanning Calorimeters (DSC). A few milligrams of the sample were weighed in a Tzero aluminum pan and sealed with a Tzero sealing cap. Heating was performed under N₂ atmosphere at a heating rate of 10 °C/min.

In the present application, thermogravimetric analysis (TGA) was measured using the following method: thermogravimetric data was collected from the sample using a thermogravimeter (TGA) of the TA Discovery series. A few milligrams of the sample was placed in a Tzero aluminum pan and heated from room temperature to the target temperature under N₂ atmosphere at a heating rate of 10 °C/minute.

It should be noted that in X-ray diffraction spectroscopy, the diffraction pattern obtained from the crystalline compound is often characteristic for a particular crystalline form, where the relative intensities of the bands (especially at low angles) may vary due to dominant orientation effects resulting from differences in crystallization conditions, particle size, and other measurement conditions. Thus, the relative intensities of the diffraction peaks are not characteristic for the crystalline form targeted, and it is the relative positions of the peaks rather than their relative intensities that should be of greater interest in determining whether they are the same as a known crystalline form. Furthermore, it is also well known in the field of crystallography that there may be a slight error in the position of the peaks for any given crystalline form. For example, the positions of the peaks can shift due to changes in temperature when analyzing the sample, sample movement, or calibration of the instrument, and the error in determination of the 2θ value is sometimes about ± 0.2°. Therefore, this error should be taken into account when determining the structure of each crystalline form. In XRD patterns, the peak position is usually expressed in terms of the 2θ angle or the interplanar spacing d, which has a simple conversion relationship: d = λ/2sinθ, where d represents the interplanar spacing, λ represents the wavelength of incident X-rays, and θ is the diffraction angle. For the same crystalline form of the same compound, the peak positions of the XRD pattern have an overall similarity, and the error in relative intensity may be large. It should also be noted that in the identification of mixtures, some of the diffraction lines are missing due to factors such as decreasing content, and in this case, there is no need to rely on the full spectrum of bands observed in the high-purity specimen, and even a single band may be characteristic for a given crystal.

It should be noted that DSC measures the transition temperature when a crystal absorbs or releases heat due to a change in its crystal structure or when the crystal melts. For the same crystalline form of the same compound, in successive analyses, the error between thermal transition temperatures and melting points are typically within about 3°C, and when it is said that a compound has a given DSC peak or melting point, it refers to that DSC peak or melting point ± 3°C. DSC provides an auxiliary method of recognizing different crystalline forms. Different crystalline forms can be recognized based on their different transition temperature characteristics.

As used herein, the term "pharmaceutical composition" refers to a formulation comprising an active compound of the present application and a carrier, excipient and/or medium generally acceptable in the art for delivering a biologically active compound to an organism (e.g., human). The pharmaceutical composition is intended to facilitate administration of the compound of the present application to an organism.

As used herein, the term "pharmaceutically acceptable inert pharmaceutical adjuvant" includes, but is not limited to, any carrier, excipient, medium, glidant, sweetener, diluent, preservative, dye/colorant, flavor enhancer, surfactant, wetting agent, dispersing agent, disintegrating agent, suspending agent, stabilizer, isotonic agent, solvent, or emulsifier, and the like, which may be used in a human or in an animal (such as a livestock).

All solvents used herein are commercially available and can be used without further purification.

In the present application, room temperature refers to 25 °C.

In the present application, low temperature refers to -10 to 25 °C.

In the present application, abbreviations have the following meanings

| | |
|---|---|
| EtOH | Ethanol |
| IPA | Isopropyl alcohol |
| NBA | n-Butanol |
| MEK | Methyl ethyl ketone |
| EA | Ethyl acetate |
| IPAc | Isopropyl acetate |
| ACN | Acetonitrile |
| THF | Tetrahydrofuran |
| MTBE | Methyl tert-butyl ether |
| Diox | 1,4-Dioxane |
| Tol | Toluene |
| DMSO | Dimethyl sulfoxide |
| Hept | n-Heptane |
| H₂O | Water |
| AC | Acetic acid |
| DVS | Dynamic Vapor Sorption |

In the present application, the content of the product was calculated as follows: content = (100% - weight loss - residue) × (100% - total impurities) * 100%.

The purity of the product was calculated by HPLC area normalization.

The following is an exemplary illustration of the content herein in conjunction with specific embodiments, but the specific examples are not intended to be any limitation on the scope herein.

### Example 1: Preparation of the Form A of the monohydrochloride of the compound of formula II

300 mg of free alkali was dissolved in 3 mL of methyl ethyl ketone/n-heptane (1:1, v/v) and a clarified solution was obtained by stirring at room temperature. To the solution was slowly added ethyl acetate solution of HCl (acid-base feeding ratio of 2:1). The mixture was stirred at room temperature for 24 h to generate a white suspension. The solid was separated by filtration, and dried under vacuum at 35 °C to obtain 285 mg of a sample of the Form A of the monohydrochloride of the compound of formula II, with a yield of about 88.6%.

### Example 2: Preparation of the Form A of the monohydrochloride of the compound of formula II

300 mg of free alkali was dissolved in 3 mL of methyl ethyl ketone/n-heptane (1:1, v/v) and a clarified solution was obtained by stirring at room temperature. To the solution was slowly added ethyl acetate solution of HCl (acid-base feeding ratio of 1.3:1). The mixture was stirred at room temperature for 24 h to generate a white suspension. The solid was separated by filtration, and dried under vacuum at 35 °C to obtain 241 mg of a sample of the Form A of the monohydrochloride of the compound of formula II, with a yield of about 75.4%.

### Example 3: Preparation of the Form B of the monohydrochloride of the compound of formula II

300 mg of free alkali was dissolved in 3 mL of ethyl acetate and a clarified solution was obtained by stirring at room temperature. To the solution was slowly added ethyl acetate solution of HCl (acid-base feeding ratio of 2:1). The mixture was stirred at room temperature for 24 h to generate a white suspension. The solid was separated by filtration, and dried under vacuum at 35 °C to obtain 223 mg of a sample of the Form B of the monohydrochloride of the compound of formula II, with a yield of about 68.4%, moisture content of 0.6%, hydrolyzed impurities 3.77%, total impurities of 4.5%, HPLC purity of 95.6%, and content of 95.2%.

### Example 4: Preparation of the Form I of the dihydrochloride of the compound of formula II

1.75 kg of free alkali was dissolved in 7 L of ethyl acetate and a clarified solution was obtained by stirring at room temperature. To the solution was slowly added ethyl acetate solution of HCl (acid-base feeding ratio of 3:1) at low temperature. The mixture was stirred at room temperature for 5 h to generate a white suspension. The solid was separated by filtration, and dried under vacuum at 40 °C to obtain 1.76 kg of a sample of the Form I of the dihydrochloride of the compound of formula II, with a yield of about 89.4%, and HPLC purity of 99.6%

### Example 5: Preparation of the Form II of the dihydrochloride of the compound of formula II

The Form I (180 mg) obtained in the example was suspended in methyl ethyl ketone (3 mL), slurried at room temperature for 24 h. The solid was separated by filtration, and dried under vacuum at 35 °C to obtain 136 mg of a sample of the Form II of the dihydrochloride of the compound of formula II, with a yield of about 75.6%.

### Example 6: Preparation of the Form III of the dihydrochloride of the compound of formula II

The Form I (180 mg) obtained in the example was suspended in methyl tert-butyl ether (3 mL), slurried at room temperature for 24 h. The solid was separated by filtration, and dried under vacuum at 35 °C to obtain 166 mg of a sample of the Form III of the dihydrochloride of the compound of formula II, with a yield of about 92.2%.

### Example 7: Rats model of deep vein thrombosis

### Experimental animals: SD rats (body weight 220 to 250 g, sex: male)

Preparation of solution: An appropriate amount of Form I was weighed, dissolved in a vehicle (0.5% (W/V) Natrosol^{™} 250 HX solution containing 0.1% (W/V) L(+)-tartaric acid) to formulate into the required concentration and then stirred uniformly, so that the drug was administered according to the predetermined dose.

Procedure: Rats were acclimatized for 3 days and randomly divided into three groups: model group, positive control group/subject group and bridging group, with 8 to 10 rats in each group. The model group was given an equivalent amount of solvent by oral gavage, the positive control group or the subject group was administered by oral gavage, and the bridging group was given the Form I of the active compound V, which was administered intravenously, and after about 45 min later, anesthesia was applied and blood was taken to start modeling. Rats in each group were anesthetized with urethane (20%, 5 mL/kg) via intraperitoneal injection. The abdominal cavity was exposed, the vena cava was bluntly separated, and two surgical sutures of 8 to 10 cm in length were placed under the blood vessels, both 1 cm apart. When the modeling time of each group was reached, rabbit thromboplastin (0.02 mg/kg) was injected into the left femoral vein, and the two sutures were knotted 10 s later, and the knotted portion of the suture was cut with ophthalmic scissors after 1 h. The thrombus was removed and weighed.

After oral administration of 2.5, 5, 10 and 20 mg/kg of Form I to rats, the blood drug levels of compound V were 190.1, 181.3, 341.9 and 609.7 ng/mL, respectively, which were basically in a dose-dependent manner, and the blood drug levels increased with the increase of the dose. At the same dose, the antithrombotic efficacy of the Form I and dabigatran etexilate was basically equivalent. The antithrombotic effect of compound V was comparable to that of oral administration of 5 mg/kg Form I after intravenous infusion of 0.1 mg/kg/h in rats, and the blood drug levels of compound V was comparable.

### Results

| Group Number | Group | Dose (mg/kg) | Thrombus weight (mg) | Thrombosis inhibition rate (%) | Blood drug level (ng/mL) |
|---|---|---|---|---|---|
| 1 | Vehicle group | N/A | 70.0±8.8 | - | |
| 2 | Dabigatran etexilate | 5 | 21.4±8.2** | 69.9 | 126.8±28.4 |
| 3 | Crystal form I group 1 | 2.5 | 51.9±8.3** | 27.0 | 190.1±62.9 |
| 4 | Crystal form I group 2 | 5 | 32.1±5.7** | 54.7 | 181.3169.5 |
| 5 | Crystal form I group 3 | 10 | 21.3±5.1** | 70.0 | 341.9±46.4 |
| 6 | Crystal form I group 4 | 20 | 14.4±3.6** | 79.7 | 609.7±174.0 |
| 7 | Compound V | 0.1 mg/kg/h | 36.8±6.6** | 48.2 | 174.2±51.8 |

| | | | | | |
|---|---|---|---|---|---|
| **P<0.01, compared with the vehicle group | | | | | |

### Example 8: Pharmacokinetic study on rats

### Experimental animals: SD rats (body weight 200 to 300 g, sex: half of male and half of female)

Preparation of solution for gavage administration: An appropriate amount of Form I was weighed, dissolved in a vehicle (0.5% (W/V) Natrosol^{™} 250 HX solution containing 0.1% (W/V) L(+)-tartaric acid) to formulate into the required concentration, stirred uniformly, and the drug was administered according to the predetermined dose.

Procedure: Rats were acclimatized for 3 days and randomly divided into groups with 8 rats in each group, with half of male and half of female. The doses of 5, 10 and 20 mg/kg were administered by gavage, and blood was collected from the jugular vein at 5 min, 10 min, 20 min, 30 min, 1 h, 2 h, 4 h, 6 h, 8 h, 12 h and 24 h after the administration of the drug, processed, and analyzed for blood drug level by LC-MS/MS.

Data were processed using Phoenix WinNonlin 6.4 non-compartmental model to calculate the pharmacokinetic parameters of rats after drug administration, including t_{1/2}, AUC₀₋ₜ, AUC_{0-∞}, Cl/F or Clₛₛ/F or Cl, V_{z}/F or Vₛₛ, MRT, Cₘₐₓ, C₅ₘᵢₙ, Tₘₐₓ, and Cₘᵢₙ, C_{avg}, and AUC_{0-τ} for multiple administrations to reach steady state, etc.

### Results:

| Dose (mg/kg) | Sex | Tₘₐₓ (h) | t_{1/2} (h) | Cₘₐₓ (ng/ml) | AUC₀₋ₜ (h·ng/mL) | AUC_{0-∞} (h·ng/mL) | V₂/F (L/kg) | Cl/F (L/h/kg) | MRT₀₋ₜ (h) | MRT_{0-∞} (h) |
|---|---|---|---|---|---|---|---|---|---|---|
| 5 | Female | 0.59±0.29 | 1.5±0.26 | 522±170 | 850+226 | 854±224 | 13.9±6.75 | 6.24±1.98 | 1.6±0.34 | 1.7+0.38 |
| | Male | 0.66±0.23 | 1.4±0.32 | 344±163 | 604±182 | 606±181 | 18.1±6.08 | 8.81±2.50 | 1.6±0.20 | 1.7±0.21 |
| | Total | 0.62±0.24 | 1.5±0.27 | 433±181 | 727±231 | 730±231 | 16.0±6.37 | 7.53±2.50 | 1.6±0.26 | 1.7±0.28 |
| 10 | Female | 0.75±0.29 | 2.6 | 1120±385 | 2060±783 | 1830 | 20.1 | 5.54 | 1.6±0.27 | 1.6 |
| | Male | 0.63±0.25 | 4.5±1.8 | 934±299 | 1460±324 | 1460±324 | 46.9±24.6 | 7.1±1.59 | 1.6±0.24 | 1.7±0.20 |
| | Total | 0.69±0.26 | 3.9±1.8 | 1030±335 | 1760±642 | 1590±346 | 38.0±23.6 | 6.58±1.53 | 1.6±0.24 | 1.7±0.26 |
| 20 | Female | 0.50±0.0 | 4.0±0.35 | 2170±380 | 3840±446 | 3850+446 | 30.8±5.67 | 5.25±0.610 | 1.7+0.19 | 1.8±0.18 |
| | Male | 0.88+0.25 | 3.7±1.7 | 2230±531 | 4650+2440 | 4660±2440 | 26.6±17.0 | 4.98±1.79 | 1.8±0.33 | 1.8±0.32 |
| | Total | 0.69±0.26 | 3.9±1.2 | 2200±429 | 4250±1680 | 4250±1680 | 28.7±11.9 | 5.12±1.25 | 1.8±0.25 | 1.8±0.25 |

The examples herein are subject to a variety of modifications and changes without departing from the scope herein. It should therefore be understood that the examples herein should not be limited to the exemplary examples described above, but should be controlled by the limitations set forth in the claims and any equivalent forms thereof.

## Claims

1. A crystalline form of a hydrochloride of a compound of formula II,

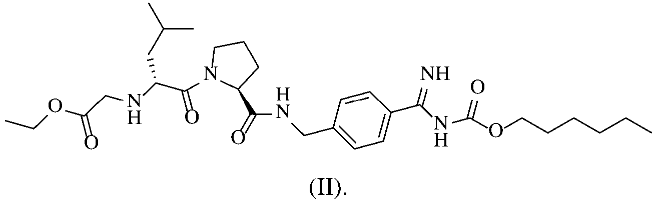

2. The crystalline form of the hydrochloride of the compound of formula II according to claim 1, wherein a stoichiometric ratio of the compound of formula II and HCl is 1:1 or 1:2.

3. A Form A of a monohydrochloride of a compound of formula II, wherein an X-ray diffraction pattern of the Form A has diffraction peaks at 2θ = 6.7 ± 0.2°, 16.2 ± 0.2°, 21.8 ± 0.2°, and 22.9 ± 0.2°; or 2θ = 6.7 ± 0.2°, 16.2 ± 0.2°, 16.5 ± 0.2°, 20.7 ± 0.2°, 21.8 ± 0.2°, and 22.9 ± 0.2°; or 2θ = 6.7 ± 0.2°, 7.8 ± 0.2°, 16.2 ± 0.2°, 16.5 ± 0.2°, 19.4 ± 0.2°, 20.7 ± 0.2°, 21.8 ± 0.2°, 22.9 ± 0.2°, 23.8 ± 0.2°, and 27.5 ± 0.2° as measured using Cu-Kα radiation.

4. The Form A according to claim 3, having an X-ray diffraction pattern as measured using Cu-Kα radiation substantially identical to that shown in FIG. 1; or
a DSC pattern substantially identical to that shown in FIG. 2; or
a TGA pattern substantially identical to that shown in FIG. 3.

5. A Form B of a monohydrochloride of a compound of formula II, wherein an X-ray diffraction pattern of the Form B has diffraction peaks at 2θ = 6.6 ± 0.2°, 15.2 ± 0.2°, 21.2 ± 0.2°, and 21.6 ± 0.2°; or 2θ = 6.6 ± 0.2°, 15.2 ± 0.2°, 20.3 ± 0.2°, 21.2 ± 0.2°, 21.6 ± 0.2°, and 23.7 ± 0.2°; or 2θ = 6.6 ± 0.2°, 9.2 ± 0.2°, 15.2 ± 0.2°, 17.6 ± 0.2°, 20.3 ± 0.2°, 21.2 ± 0.2°, 21.6 ± 0.2°, 22.4 ± 0.2°, 23.7 ± 0.2°, and 25.7 ± 0.2° as measured using Cu-Kα radiation.

6. The Form B according to claim 5, having an X-ray diffraction pattern as measured using Cu-Kα radiation substantially identical to that shown in FIG. 4; or
a DSC pattern substantially identical to that shown in FIG. 5; or
a TGA pattern substantially identical to that shown in FIG. 6.

7. A dihydrochloride of a compound of formula II;

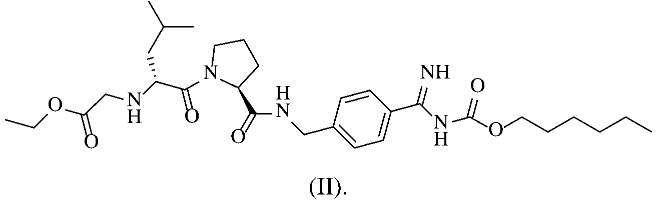

8. A Form I of a dihydrochloride of a compound of formula II, wherein an X-ray diffraction pattern of the Form I has diffraction peaks at 2θ = 6.0 ± 0.2°, 17.1 ± 0.2°, 19.9 ± 0.2°, and 23.3 ± 0.2°; or 2θ = 6.0 ± 0.2°, 17.1 ± 0.2°, 19.9 ± 0.2°, 23.3 ± 0.2°, 25.4 ± 0.2°, and 25.7 ± 0.2°; or 2θ = 6.0 ± 0.2°, 13.1 ± 0.2°, 17.1 ± 0.2°, 17.5 ± 0.2°, 19.9 ± 0.2°, 20.2 ± 0.2°, 22.0 ± 0.2°, 23.3 ± 0.2°, 25.4 ± 0.2°, and 25.7 ± 0.2° as measured using Cu-Kα radiation.

9. The Form I according to claim 8, having an X-ray diffraction pattern as measured using Cu-Kα radiation substantially identical to that shown in the Form I of FIG. 7; or
a DSC pattern substantially identical to that shown in FIG. 8; or
a TGA pattern substantially identical to that shown in FIG. 9.

10. A Form II of a dihydrochloride of a compound of formula II, wherein an X-ray diffraction pattern of the Form II has diffraction peaks at 2θ = 6.0 ± 0.2°, 17.1 ± 0.2°, 20.0 ± 0.2°, and 22.8 ± 0.2°; or 2θ = 6.0 ± 0.2°, 13.4 ± 0.2°, 17.1 ± 0.2°, 20.0 ± 0.2°, 21.2 ± 0.2°, and 22.8 ± 0.2°; or 2θ = 6.0 ± 0.2°, 9.9 ± 0.2°, 13.4 ± 0.2°, 17.1 ± 0.2°, 20.0 ± 0.2°, 20.5 ± 0.2°, 21.2 ± 0.2°, 22.8 ± 0.2°, 26.9 ± 0.2°, and 30.2±0.2° as measured using Cu-Kα radiation.

11. The Form II according to claim 10, having an X-ray diffraction pattern as measured using Cu-Kα radiation substantially identical to that shown in the Form II of FIG. 7; or
a DSC pattern substantially identical to that shown in FIG. 10; or
a TGA pattern substantially identical to that shown in FIG. 11.

12. A Form III of a dihydrochloride of a compound of formula II, wherein an X-ray diffraction pattern of the Form III has diffraction peaks at 2θ = 6.0 ± 0.2°, 17.2 ± 0.2°, 20.1 ± 0.2°, and 22.0 ± 0.2°; or 2θ = 6.0 ± 0.2°, 13.5 ± 0.2°, 17.2 ± 0.2°, 20.1 ± 0.2°, 21.2 ± 0.2°, and 22.0 ± 0.2°; or 2θ = 6.0 ± 0.2°, 9.3 ± 0.2°, 13.5 ± 0.2°, 17.2 ± 0.2°, 18.7 ± 0.2°, 20.1 ± 0.2°, 22.0 ± 0.2°, 21.2 ± 0.2°, 24.0 ± 0.2°, and 26.4 ± 0.2° as measured using Cu-Kα radiation.

13. The Form III according to claim 12, having an X-ray diffraction pattern as measured using Cu-Kα radiation substantially identical to that shown in the Form III of FIG. 7; or
a DSC pattern substantially identical to that shown in FIG. 12; or
a TGA pattern substantially identical to that shown in FIG. 13.

14. A preparation method for the Form A according to claim 3 or 4, comprising following steps:
(a) dissolving a free alkali of the compound of formula II in a mixed solvent of methyl ethyl ketone/n-heptane and stirring to obtain a clarified solution; and
(b) adding slowly a solution of HCl in ethyl acetate to the clarified solution, stirring, filtering, and drying;
an acid-base feeding ratio of the HCl and the free alkali of the compound of formula II is 1:1 to 2:1, preferably 1.1:1 to 1.5:1;
preferably, in step (a), dissolving the free alkali of the compound of formula II at room temperature, and in step (b), stirring at room temperature for 24 h.

15. A preparation method for the Form B according to claim 5 or 6, comprising following steps:
(a) dissolving a free alkali of the compound of formula II in ethyl acetate and stirring to obtain a clarified solution; and
(b) adding slowly a solution of HCl in ethyl acetate to the clarified solution, stirring, filtering, and drying;
an acid-base feeding ratio of the HCl and the free alkali of the compound of formula II is 1:1 to 2:1, preferably 1.1:1 to 1.5:1;
preferably, in step (a), dissolving the free alkali of the compound of formula II at room temperature, and in step (b), stirring at room temperature for 24 h.

16. A preparation method for the Form I according to claim 8 or 9, comprising following steps:
(a) dissolving a free alkali of the compound of formula II in ethyl acetate and stirring to obtain a clarified solution; and
(b) adding slowly a solution of HCl in ethyl acetate to the clarified solution, stirring, filtering, and drying;
an acid-base feeding ratio of the HCl and the free alkali of the compound of formula II is 2:1 to 5:1, preferably 2.5:1 to 4:1;
preferably, in step (a), dissolving the free alkali of the compound of formula II at room temperature, and in step (b), stirring at low temperature for 5 h.

17. A preparation method for the Form II according to claim 10 or 11, comprising following steps: suspending a Form I of the dihydrochloride of the compound of formula II in a ketone solvent, slurrying, and filtering and drying;
preferably, the ketone solvent is selected from the group consisting of acetone, butanone, methyl ethyl ketone, and combinations thereof;
preferably, the slurrying is performed at a temperature from room temperature to 50 °C for 24 h.

18. A preparation method for the Form III according to claim 12 or 13, comprising following steps: suspending a Form I of the dihydrochloride of the compound of formula II in methyl tert-butyl ether, slurrying, and filtering and drying;
preferably, the slurrying is performed at a temperature from room temperature to 50 °C for 24 h.

19. A pharmaceutical composition comprising the crystalline form of the hydrochloride of the compound of formula II according to any one of claims 1-2, or the monohydrochloride and/or the dihydrochloride of the compound of formula II according to any one of claims 3 to 13, together with one or more pharmaceutically acceptable inert pharmaceutical adjuvant(s);
preferably, the pharmaceutical composition is formulated for oral administration.

20. A pharmaceutical composition comprising 95% or more of a dihydrochloride of a compound of formula II.

21. A crystal composition comprising 80% or more of a Form I of a hydrochloride of a compound of formula II.

22. Use of the crystalline form of the hydrochloride of the compound of formula II according to claim 1 or 2, the monohydrochloride or the dihydrochloride of the compound of formula II according to any one of claims 3 to 13, the pharmaceutical composition according to claim 19 or 20, or the crystal composition according to claim 21 in the manufacture of a medicament for the prevention and treatment of a thrombin-mediated and/or thrombin-related disease.

23. Use of the crystalline form of the hydrochloride of the compound of formula II according to claim 1 or 2, the monohydrochloride or the dihydrochloride of the compound of formula II according to any one of claims 3 to 13, the pharmaceutical composition according to claim 19 or 20, or the crystal composition according to claim 21 in the manufacture of a medicament for the treatment of a venous and/or arterial thrombotic disease.
